# EUROPEAN PATENT SPECIFICATION

(11) **EP 2 075 017 B1**
(45) Date of publication and mention of the grant of the patent: **13.04.2011**
(21) Application number: 07425823.7
(22) Date of filing: 24.12.2007
(51) Int. Cl.: A61M 1/00, F16L 37/26, G01L 19/00

(54) **Suction assembly for use in medical field**
Medizinische Saugeinrichtung
Dispositif de suction à usage médical

(43) Date of publication of application: 01.07.2009
(73) Proprietor: Flow Meter S.p.a., 24040 Levate (Bergamo) (IT)
(72) Inventor: Paratico, Roberto, 24040 Levate (Bergamo (IT)
(74) Representative: Botti, Mario

(56) References cited:
- EP-A- 0 047 351
- EP-A- 0 997 680
- WO-A-93/25248
- US-A- 4 744 785
- US-A- 4 784 175
- US-B1- 6 342 048

## Description

### Field of application

The present invention refers in its widest general aspect to the field of vacuum distribution plants, preferably but not exclusively in the medical field, and in particular to a suction assembly of fluids, preferably organic fluids, for suctioning such fluids from a patient.

The suction assembly is of the type comprising a regulation device intended to be connected to a vacuum source, and a safety jar for collecting fluids, wherein said safety jar is fastened in a removable manner to the regulation device.

A suction assembly of this type is known from WO-A-93/25248.

### Prior Art

With reference to the above-mentioned field, the need to connect in a secure manner a safety jar for collecting fluids to the regulation device is known. In particular, such safety jar has the purpose of guaranteeing proper operation of the vacuum regulation in the device. In fact, such safety jar is interposed in fluid connection between the regulation device and a primary container for organic fluids, the latter connected to a patient, to guarantee that in case the fluid in the primary container fills-up exceeding a given level, or threshold, such fluid does not damage the regulation device and the hospital circulation system or the vacuum source.

With this aim, the safety jar is provided with an entrance mouth facing upwards, connected in a removable manner to the regulation device and provided with an overflow valve, and a side mouth for the connection, through a flexible pipe, with the primary container of organic fluids.

In a known suction assembly, the removable connection is obtained by means of a screw connection, and for such purpose a male threading is provided for on the external surface of a connection mouth of the regulation device, and a corresponding female threading is provided for on the internal surface of the entrance mouth of the safety jar.

Though advantageous from different aspects, and substantially meeting the objective, however the known device has some drawbacks yet to be overcome up to date.

In particular, the main drawback depends on the fact that the safety jar must be easy to connect or remove from the regulation device for cleaning or disinfection, so as to eliminate the risk of accidental or cross contamination.

Another drawback lies in the fact that the screw connection is not quick to perform, and it does not even allow to control, upon completion of the screwing operations, neither the angular position of the safety jar with respect to the regulation device and more in particular the angular position of the side mouth for the connection with the primary container of the organic fluids, nor the airtight fastening, the latter being an essential condition to guarantee an efficient suctioning. The first of the last two drawbacks, is particularly disadvantageous in case the suction assembly is fastened directly onto an end unit arranged on the wall of the vacuum distribution plant, and in particular the side mouth, upon completion of the screwing operations located too close to the wall, and thus difficult to access.

Thus, the technical problem on which this invention is based is that of providing a suction assembly of the type mentioned beforehand, capable of overcoming the above-mentioned drawbacks, related to the known art, and in particular allow to obtain a univocal, secure and quick connection of the safety jar to the regulation device.

### Summary of the invention

The above-mentioned technical problem is solved by a suction assembly of the previously indicated type, which according to the invention comprises a bayonet clutch for fastening in a removable manner the safety jar to the regulation device.

The main advantage of the suction assembly according to the present invention lies in that a bayonet clutch guarantees quick, safe and accurate locking of the safety jar to the regulation device. As a matter of fact, a bayonet clutch comprises a fixed part and a movable part whose fastening, and whose loosening, require a predetermined operation to be performed, thus hindering, in particular, loosening of the parts unless explicitly performed by the authorised personnel.

According to the invention, the fixed part of the bayonet clutch is formed by a connection mouth of the regulation device, and the moveable part is formed by a locking ring associated, in a rotatably idle manner, to an entrance mouth of the safety jar. Basically, the bayonet clutch comprises a locking ring, which acts as a locking element, which is out of rigid engagement with the safety jar; hence, in case of inadvertent movement of the safety jar, the connection with the regulation device remains intact.

Furthermore, thanks to the fastening obtained by means of the locking ring rotating in an idle manner, the safety jar can be fixed onto the regulation device at a desired predetermined angular position, which does not depend on the fastening operation.

Preferably, the connection mouth of the regulation device is provided with teeth or protrudes, while the locking ring and the entrance mouth of the safety jar are provided on the respective edge with recesses which, once aligned with each other, allow an insertion of the teeth of the connection mouth of the regulation device.

In particular, the locking ring is formed by a cylindrical collar provided at its end, on the side facing the regulation device, with an edge section bent towards the inner zone, or the centre, of the collar, and in which the above-mentioned opposite recesses are provided. Due to such configuration of the collar, with a bent edge and recesses, an L-shaped groove is obtained on the locking ring.

Thus, the teeth of the regulation device are inserted into the recesses of the locking ring and accommodated in the recesses of the jar entrance mouth and, upon an angular movement of the locking ring around the jar entrance mouth, they are locked by the bent edge of the locking ring. Preferably, the suction assembly is provided with indicator marks to indicate visually, respectively the locked position and the unlocked position, of the locking ring and snap-locks for establishing the angular position of the locking ring with respect to the safety jar at the two lock and unlock positions.

In a preferred embodiment, the locking ring is axially held on the entrance mouth of the safety jar by means of interposition of a annular element, which rotates jointly with the locking ring.

Furthermore, the annular element has a substantially C-shaped cross-section and it is provided, on one side, with a recess coupled with a circular projection protruding from the external surface of the entrance mouth of the safety jar, and on the other it is provided, on its external surface, with opposite teeth, which are snap-fitted into the corresponding openings provided for on the surface of the locking ring. Preferably, the ring is formed by two half-rings to facilitate the relative assembly onto the safety jar.

In this last embodiment, the above-mentioned snap-locks are provided between the annular element, which as mentioned rotates jointly with the locking ring, and the safety jar and they are formed by a pair of small protrudes which snap-fit, with a "click" sound, into corresponding seats provided on the entrance mouth of the safety jar.

Further characteristics and advantages of the suction assembly according to the invention shall be observed from the description of one of its embodiments outlined hereinafter provided for exemplifying and non-limiting purposes with reference to the drawings attached.

### Brief description of the drawings

In such drawings:

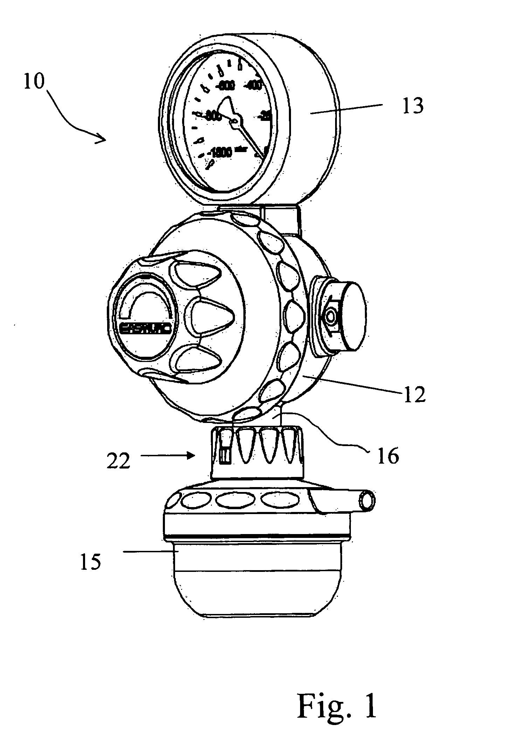

Figure 1 is an axonometric view of the suction assembly according to invention.

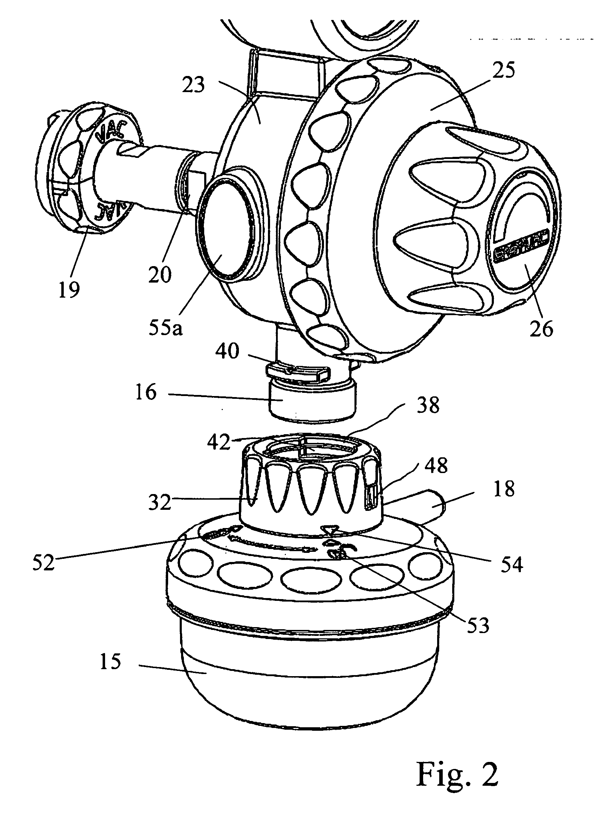

Figure 2 is an enlarged view of a detail of the assembly of figure 1 with the parts detached.

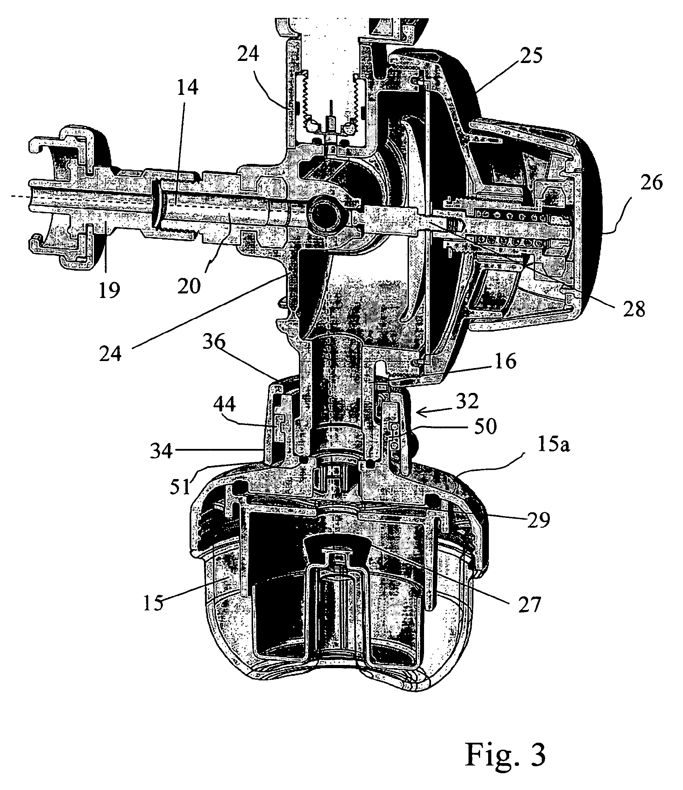

Figure 3 is an enlarged scale cross-section of the device of figure 1.

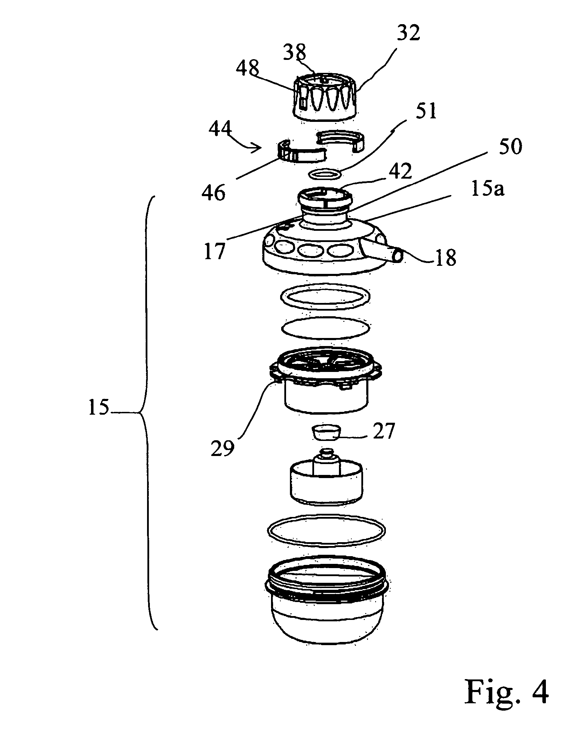

Figure 4 is a view of a safety jar according to the invention, with the parts detached, of the device of figure 1.
Figure 5 is a top view of the safety jar of figure 4.
Figure 6 is a view with the parts detached of the regulation device according to the invention.
Figure 7 is a cross-section of a detail of the regulation device of figure 6.
Figure 8 is a cross-sectional view with the parts detached of the detail of figure 7.
Figure 9 is a rear view of another detail of the assembly of figure 1.
Figure 10 is a rear view with parts detached of the detail of figure 9.
Figure 11 is a view of the regulation device as an alternative to the device of figure 6.

### Detailed description of a preferred embodiment

With reference to such figures, a suction assembly according to the invention adapted to be connected to a vacuum source (not illustrated), for the suction of fluids, in particular organic fluids is indicated in its entirety and schematically by 10.

The assembly is of the type comprising a regulation device 12, provided with a connection mouth 16, a safety jar 15 provided with an entrance mouth 17 in fluid connection with the connection mouth 16 of the regulation device 12, and removable fastening means 22 for connecting the entrance mouth 17 of the safety jar 15 with the connection mouth 16 of the regulation device 12.

Provided in assembly 10 is a suction path 14, indicated with a dashed lined in the drawings, extended between a first mouth 18 projecting sideways from the safety jar 15 and adapted to be connected, through a flexible pipe, to a primary container for the collection of liquids to be suctioned (not illustrated), and a second mouth 20 associated to the regulation device 12 and adapted to be connected with the vacuum source.

As observable from the drawings, the second mouth 20 is provided with an engagement device 19 for connecting the device 10 with an end unit (not visible in the drawings) of a circulation system connected to the vacuum source.

The regulation device 12 is basically formed by a valve comprising a cylindrical shaped valve body and formed by a mantle 23, a bottom wall 24, and a cover 25. The cover 25 is provided with an operating knob 26 cooperating with a sliding element 28 for regulating the vacuum. The device 10 is completed by a vacuum gauge 13, connected in a known manner to the regulation device 12. It should be understood that the vacuum regulation in the regulation device 12 and the measurement of the vacuum by means of the vacuum gauge 13 were performed according to an entirely known manner, which do not relate to the present invention, and thus shall not be described. Also the safety jar 15 comprises within it components of the known type, such as an overflow valve 27 and a filter 29, which shall not be described.

As can be seen from the drawings, the connection mouth 16 of the regulation device 12 is formed by a piece of pipe projecting towards the lower part of the mantle 23, and it is connected to the safety jar 15 which is arranged beneath the regulation device 12. Also the entrance mouth 17 of the safety jar 15 is tubular shaped, and it is formed by an appendage projecting upwards from a cover 15a of the safety jar 15.

According to the invention, the removable fastening means 22 connecting the regulation device 12 to the safety jar 15 comprise a bayonet clutch, that is a coupling formed by two parts one of which is a fixed part and the other a movable part, in which the movable part moves and rotates with respect to the fixed part, for the connection of the two parts, and subsequently rotates and moves, for loosening.

In this case, the fixed part is formed by a connection mouth 16 of the regulation device 12, and the moveable part is formed by a locking ring 32 associated in a rotatably idle manner to the entrance mouth 17 of the safety jar 15. In particular, the locking ring 32 comprises a cylindrical collar 34 provided with, at its top, that is on the side facing the regulation device 12, an edge 36, which is bent towards the centre of the cylindrical collar 34 itself, and on which two opposite recesses 38 are provided for. Due to this configuration of the edge and the recesses, at the two recesses 38, defined in the locking ring 32 is a substantially L-shaped groove.

Also the entrance mouth 17 is provided at its top edge with two opposite recesses 42 and having the same dimensions of the two recesses 38 of the locking ring 32.

The connection mouth 16, which as mentioned is formed by a piece of pipe which integrally projects from the mantle 23 of the regulation device 12, is provided on its external surface with two opposite teeth 40, or protrudes.

Both the recesses 38 provided for on the bent edge of the locking ring 32 and the recesses 42 provided for on the edge of the entrance mouth 17 of safety jar 15, have shapes and dimensions substantially corresponding to the teeth 40 of the regulation device 12, and in particular they are dimensioned to accommodate with a limited clearance the opposite teeth 40 of the regulation device 12.

In order to axially hold the locking ring 32 on the entrance mouth 17 of the jar 15, the device 10 comprises an annular element 44 interposed between the locking ring 32 and the entrance mouth 17, the annular element rotating jointly with the locking ring 32. The annular element 44 is provided with a substantially C-shaped cross-section, and it is fastened to the internal surface of the locking ring 32 through snap-fitting, obtained by means of elastic deformation, of opposite teeth 46, projecting from the external surface of the annular element 44, into corresponding openings 48 provided for on the cylindrical surface of the locking ring 32. Furthermore, the annular element 44 is fixed to the entrance mouth 17 through the coupling of an internal recess with a circular projection 50 integrally projecting from the external surface of the entrance mouth 17.

The snap-fitting of the teeth 46 can be visually controlled through the two opposite openings 48 of the locking ring.

Preferably, the annular element 44 is formed by two half-rings joined to each other through engagement and centring pins.

The connection between the safety jar 15 and the regulation device 12, is performed as follows.

Initially the two half-rings forming the annular element 44 are closed on the circular projection 50 of the entrance mouth 17, in such a manner that the annular element 44 is axially held on the entrance mouth 17.

The locking locking ring 32 is fitted onto the entrance mouth 17 until snap-coupling of the external teeth 46 of the annular element 44 into the openings 48 of the locking ring 32 is obtained. The locking ring 32 which rotates jointly with the ring 44, is then rotated around the entrance mouth 17 until the recesses 38, and 42 respectively, are aligned to each other. In this position, the locking ring 32 is arranged at the so called unlocked condition.

The connection mouth 16 of the regulation device is inserted into the entrance mouth 17 of the jar 15, until the respective teeth 40 abut, thus they are accommodated, at the bottom of the recesses 42 of the safety jar 15.

The locking ring 32 is subsequently rotated around the axis of the entrance mouth 17 by approximately 90°, in such a manner to position the bent edge 36 of the locking ring 32 above the teeth 40. Thus a locked condition is obtained. In this condition, the end of the connection mouth 16 of the regulation device 12 is forced by the locking ring 32, it determining a pressing condition, against a sealing ring 51 provided for inside the entrance mouth 17.

In order to make the locked and unlocked conditions of the regulation device 12 visible from the external with respect to the safety jar 15, on the external surface of the safety jar, in the proximity of the locking ring 32, two reference marks are indicated, one 52 for locking identified by a closed padlock, the other 53 for unlocking, identified by an open padlock, respectively provided at an angular distance of 90° approximately.

Provided on the external surface of the locking ring 32, is an indicator mark 54, intended to be provided alternatively to the two reference marks, of locked 52 and unlocked conditions, drawn on the surface of the safety jar 15.

Furthermore, provided are snap-locks for stabilising the angular position of the locking ring 32 respectively at the locked or unlocked condition. In particular, the aforementioned snap-locks are provided between the annular element 44, which as mentioned rotates jointly with the locking ring, and the safety jar 15, and they are formed by a pair of small protrudes (not visible in the drawings) which snap-lock, with a "click" sound, into connected seats provided on the circular projection 50 of the entrance mouth 17 of the safety jar 15 in the two locked or unlocked positions.

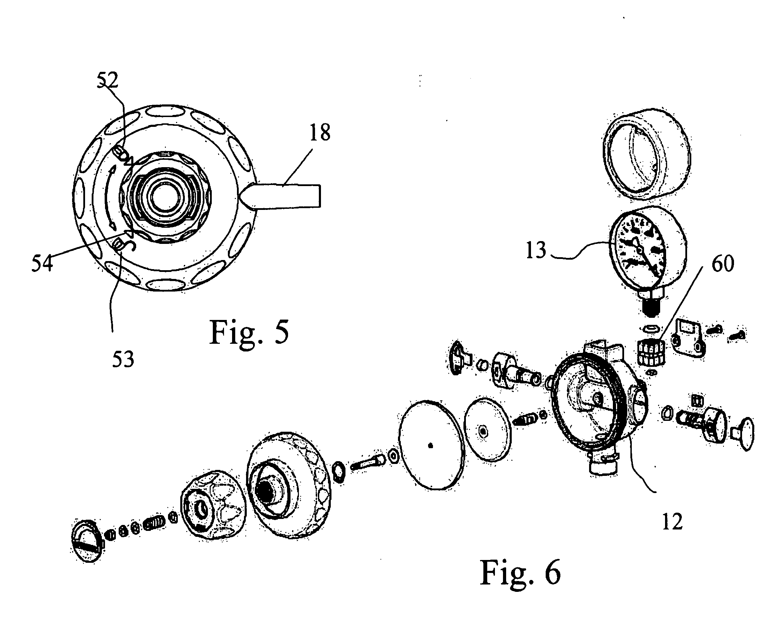

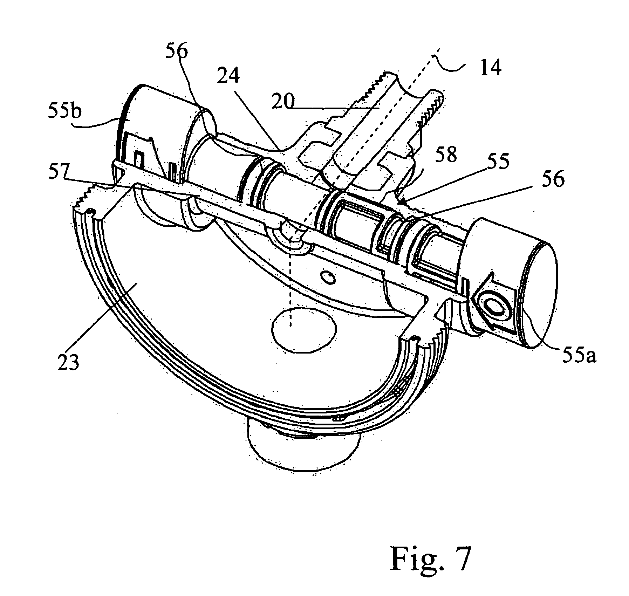

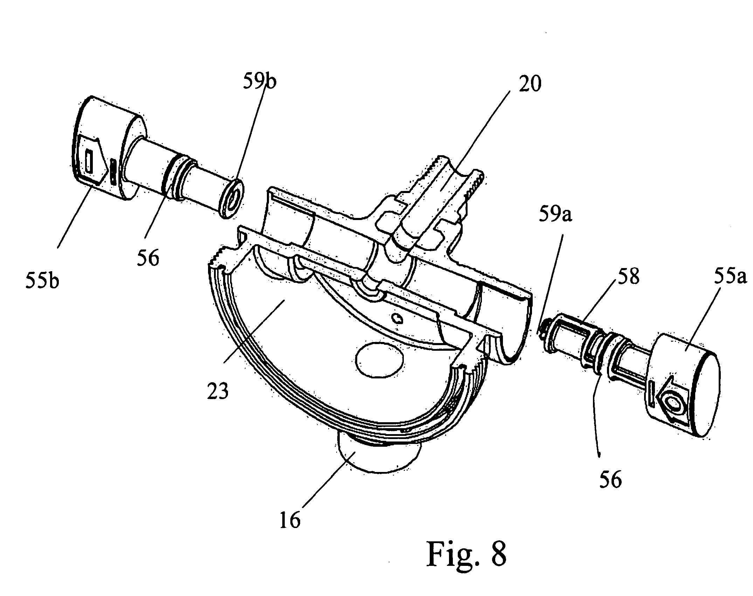

With reference to figures 6, 7 and 8, illustrated is a further aspect of the suction assembly 10. In particular, the regulation device 12 comprises a second sliding element 55, which acts as a switch I-0, respectively intended for opening and closing, the suction path 14. The sliding element 55, provided with sealing rings 56, is substantially tubular shaped, and it is accommodated in sliding manner into a corresponding cylindrical shaped housing 57 provided in the regulation device 12. In particular, such housing 57 extends in a transverse manner in the valve body near the second mouth 20. The sliding element 55 can be moved in a linear manner between a first closure position (OFF or 0) in which the sliding element 55 closes the suction path 14 near the second mouth 20, and a second open position (ON or I) in which the switch leaves the suction path 14 free. In the solution illustrated, in order to obtain the closure of the suction path 14, the switch bears , on its external surface, a rubber plug 58 which at closure position blocks the suction path 14 in a airtight manner at the second mouth 20.

In order to move the sliding element 55 between the two positions all that is required is to push the sliding element 55 to the right or to the left of its opposite ends. In particular, the opposite ends are provided with buttons 55a and 55b, one 55a red and indicating the closure OFF or 0, the other 55b green and indicating the open condition ON or I. The sliding element 55 is dimensioned in such a manner that once arranged in the housing 57 of the regulation device 12 only one of the two ends/button 55a, 55b projects from the mantle 23. In this manner, by pressing, in an alternating manner, from opposite sides, one or the other button end 55a, 55b it is possible to interrupt or restore suction. Preferably, this sliding element 55 is made in two pieces, and in particular substantially tubular shaped pieces, having respective adjacent ends which are connected through snap-coupling. In particular, a first piece of the sliding element 55 is provided with an end section bearing two opposite front teeth 59a. A second piece of the sliding element 55 is provided with an end section 59b shaped to form a piece of pipe, inside which the front teeth 59a of the other piece are fitted by means of an elastic engagement.

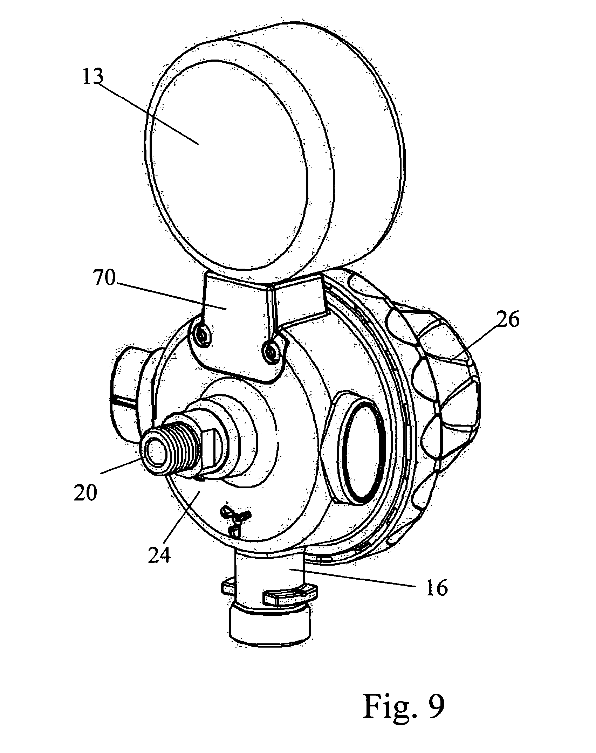

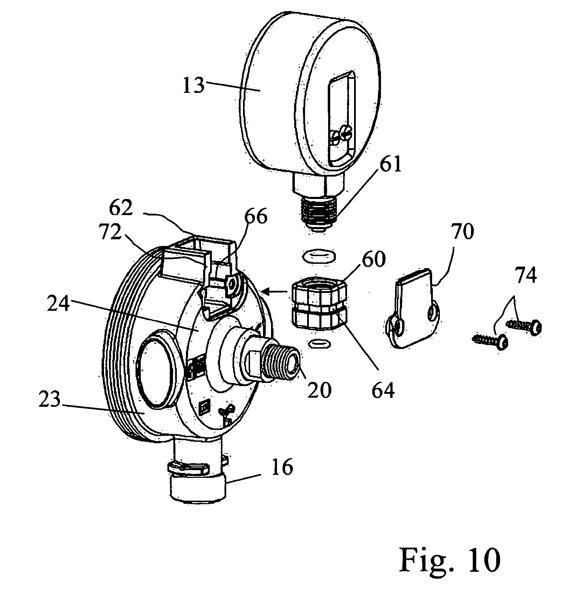

With reference to figures 9 and 10 illustrated is another aspect of the suction assembly 10 according to the present invention, which regards the connection between the regulation device 12 and the vacuum gauge 13.

In particular, the vacuum gauge 13 comprises a drawer 60 movable in a transverse manner in the body of the regulation device 12 from the rear, that is from the rear part behind the bottom wall 24 of the regulation device 12, as indicated by the arrow, and it can be accommodated into a housing 62 provided in the regulation device 12 itself.

The drawer 60 is fastened by means of a threaded connection 61 onto the lower end of the vacuum gauge 13.

In particular, the drawer 60 is formed by a substantially block-shaped body and provided on its external surface with a substantially horizontal perimeter groove 64. The housing 62 of the regulation device 12 is provided with corresponding horizontal ribs 66 which can be fitted into the groove 64 of the drawer 60, to allow a guided insertion of the latter into the housing 62.

Furthermore, provided is a covering lid 70 which can be fitted from above along connected guides 72 provided on the opposite sides of the housing 62 on the bottom wall 24 of the body of the regulation device 12, and therein fixed by means of screws 74.

The main advantage of the invention lies in the fact that, due to the bayonet clutch, a safe, quick and accurate connection between safety jar 15 and the regulation device is guaranteed.

The connection is made safer by the fact that the locking ring, which acts as a locking element, is disengaged with respect to the safety jar. Therefore, in case of inadvertent impact or stroke on the safety jar, due to the locking ring disengaged with respect to it, the connection is secured.

In fact, to remove the lock between the regulation device and the safety jar, the locking ring requires to be moved angularly, between the above-mentioned locked position and the above-mentioned unlocked position, independently with respect to the safety jar.

Additionally, thanks to the locking ring, the safety jar can be fastened in such a manner that the side mouth for connection with the primary container for the organic fluids is at a preferred predetermined angular position.

It should be understood that, within the scope of the present invention, a connection by means of a bayonet clutch between the regulation device and the safety jar can be provided, in a dual or inverted solution with respect to the one described up to this point, that is the solution in which the fixed part is formed by the entrance mouth of the safety jar and the moveable part is formed by the same locking locking ring described above, which is associated in a rotatably idle manner to the connection mouth of the regulation device. In this dual solution the entrance mouth of the jar is provided with the above-mentioned teeth, while the connection mouth of the regulation device is provided with recesses for coupling with the teeth.

It is clear that all the above-mentioned advantages are obtained also in the dual solution mentioned above, precisely to the fact that, even in this case the, the locking ring is out of a rigid engagement with the safety jar.

A further advantage lies in the possibility of visualising the unlocked and locked positions between the locking ring and the regulation device, and such characteristic is particularly useful when the regulation device is fixed to the jar, and the position regarding the recesses of the safety jar and of the locking ring is not visible from the external and an easy alignment for unlocking would not be easy to obtain, unless upon several attempts.

The locking or unlocking characteristic is further facilitated by the coupling by means of the "click" sound of the snap-locks between the annular element and the entrance mouth of the safety jar.

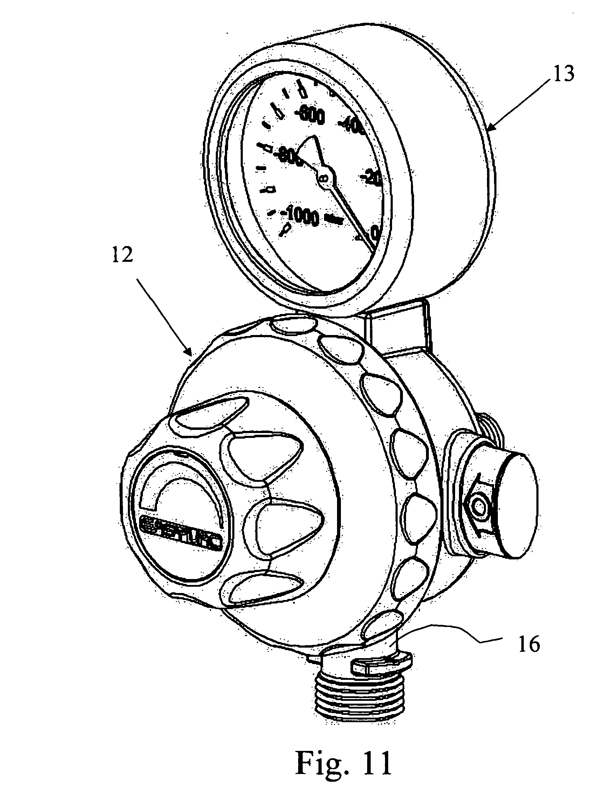

Another advantage lies in the fact that the connection mouth of the regulation device is formed by a substantially tubular appendage which projects from the mantle of the regulation device. Due to this shape, it is possible to use a regulation device, similar to the one of the known art provided with a tubular appendage bearing an external threading, and thus already available in the market, which, as illustrated in figure 11, with respect to the known art, is provided with protrudes for the bayonet clutch. In other words the external threading does not prevent fastening with the safety jar through bayonet clutch.

Another advantage lies in the elastic coupling configuration by means of the above-mentioned annular element between the locking ring and the entrance mouth of the safety jar, which facilitates assembly and also allows to ease the mechanical stress, to which especially the safety jar is subjected to.

Another advantage lies in the exceptional simplicity of assembly of all the components mentioned above of the device according to the invention.

Particularly advantageous is also the fact that the above-mentioned annular element is made in two pieces, thus facilitating assembly.

Also regarding the sliding element which acts as a switch made in two pieces, it can be observed that the two pieces can be coupled reciprocally inside the regulation device, by simply fitting them from opposite sides and snap-coupling the respective adjacent ends without necessarily removing the knob and the cover of the body of the regulation device.

A similar advantage is offered by the drawer for the connection of the vacuum gauge which is fitted in a transverse manner from the rear of the regulation, without requiring removal or opening of the latter.

Obviously, with the objective of meeting required and specific needs, a man skilled in the art may perform on the suction assembly described above numerous modifications and variants, all falling within the scope of protection of the invention as defined by the following claims.

## Claims

1. Suction assembly for use in medical field adapted to be connected to a vacuum source for the suction of fluids, in particular organic fluids, of the type comprising a regulation device (12) intended to be connected to a vacuum source, and a safety jar (15) for collecting fluids, wherein said safety jar (15) is fastened in a removable manner to the regulation device (12) by a bayonet clutch (30), **characterised in that** the bayonet clutch (30) is provided with a fixed part formed by a connection mouth (16) of the regulation device (12), and a moveable part formed by a locking ring (32) arranged in a rotatably idle manner on an entrance mouth (17) of the safety jar (15).

2. Suction assembly according to claim 1, **characterised in that** the regulation device (12) comprises a cylindrical shaped body, formed by a mantle (23), a bottom wall (24), and a closure cover (25).

3. Suction assembly according to claim 2, **characterised in that** the connection mouth (16) of the regulation device (12) is formed by a piece of pipe projecting from the regulation device (12).

4. Suction assembly according to claim 2, **characterised in that** the entrance mouth (17) of the safety jar (15) is tubular shaped, and it is formed by an appendage projecting from the safety jar (15).

5. Suction assembly according to any one of the preceding claims 1 to 4, **characterised in that** the locking ring (32) comprises a cylindrical collar (34) provided with, on the side facing the regulation device (12), an edge (36) bent towards the centre of the cylindrical collar (34) itself, on which two opposite recesses (38) are provided.

6. Suction assembly according to claim 5, **characterised in that** the entrance mouth (17) is provided at an end with two opposite recesses (42), and having the same dimensions of the two recesses (38) of the locking ring (32).

7. Suction assembly according to claim 5 or 6, **characterised in that** the connection mouth (16) of the regulation device (12) is provided on its external surface with two opposite teeth (40), and **in that** both the recesses (38) provided on the bent edge of the locking ring (32) and the recesses (42) provided on the edge of the entrance mouth (17) of the safety jar have shapes and dimensions substantially corresponding to the teeth (40) of the regulation device (12), and in particular they are dimensioned to accommodate with limited clearance the opposite teeth (40) of the regulation device (12).

8. Suction assembly according to claim 1, **characterised in that** it comprises a annular element (44), which rotates jointly with the locking ring (32), and is interposed between the locking ring (32) and the entrance mouth (17) of the safety jar (15), for axially holding the locking ring (32) with respect to the entrance mouth(17).

9. Suction assembly according to claim 8, **characterised in that** the annular element (44) is connected to the locking ring (32) by means of snap-fitting, obtained through elastic deformation, of teeth (46) projecting from the external surface of the annular element (44) into corresponding openings (48) provided on the cylindrical surface of the locking ring (32).

10. Suction assembly according to claim 9, **characterised in that** the annular element (44) has a substantially C-shaped cross-section comprising a recess, and it is connected to the entrance mouth (17) by means of coupling the recess with a corresponding circular projection (50) integrally projecting from the external surface of the entrance mouth (17).

11. Suction assembly according to claim 10, **characterised in that** the annular element (44) is formed by two half-rings joined to each other by means of coupling and centring pins.

12. Suction assembly according to claim 1, **characterised in that** on the external surface of the safety jar (15) indicated in the proximity of the locking ring (32) are reference marks, one (52) for locking, the other (53) for unlocking, respectively arranged at a predetermined angular distance.

13. Suction assembly according to claim 12, **characterised in that** provided on the external surface of the locking ring (32) is an indicator mark (54) intended to be arranged alternatively at reference marks, lock and unlock, drawn on the surface of the safety jar (15).

14. Suction assembly according to any of the preceding claims, **characterised in that** it comprises snap-locks for stabilising the angular position of the locking ring at the two lock and unlock positions.

15. Suction assembly according to any of the preceding claims, **characterised in that** it comprises a sliding element (55) serving as a switch intended for respectively opening and closing a suction path (14) extended in the regulation device (12), said sliding element (55) substantially being tubular shaped, and being accommodated in a sliding manner in a corresponding substantially cylindrical shaped housing (57) provided in the regulation device (12).

16. Suction assembly according to claim 15, **characterised in that** the sliding element (55) has opposite ends (55a, 55b) shaped to form a button, and it is dimensioned in such a manner that once arranged in the housing (57) of the regulation device (12) only one of the two ends (55a, 55b) projects from the regulation device (12).

17. Suction assembly according to claim 15 or 16, **characterised in that** the sliding element (55) is made in two pieces, and in particular two substantially tubular shaped pieces, and having respective adjacent ends connected through snap-coupling.

18. Suction assembly according to claim 17, **characterised in that** a first part of the sliding element (55) is provided with an end section bearing two opposite front teeth (59a) and a second piece of the sliding element is provided with an end section (59b) shaped to form a piece of pipe, inside which the front teeth (59a) of the other piece are fitted by means of an elastic engagement.

19. Suction assembly according to any of the preceding claims, **characterised in that** it further comprises a vacuum gauge (13) connected to the regulation device (12) and means for connecting the vacuum gauge (13) to the regulation device (12) wherein said means comprise a drawer (60) moveable in a transverse manner in the regulation device (12) and can be accommodated into a housing (62) provided for in the regulation device (12) itself.

20. Suction assembly according to claim 19, **characterised in that** the drawer (60) is fixed by means of a threaded connection (61) to a lower side of the vacuum gauge (13).

21. Suction assembly according to claim 20, **characterised in that** the drawer (60) is formed by a body substantially shaped to form a block which can be fitted in a guided manner into the housing (62).

22. Safety jar for collecting organic fluids for a suction assembly intended for medical field, comprising an entrance mouth (17), intended to be fastened in a removable manner to a vacuum regulation device (12), and a connection mouth (18) intended to be connected to the fluids to be suctioned, **characterised in that** it comprises a locking ring (32) for fastening in a removable manner the regulation device (12), said locking ring (32) being placed in a rotatably idle manner on the entrance mouth (17), and constituting a movable part of a bayonet clutch (30) to be fastened onto a corresponding fixed part of the bayonet clutch associated to the regulation device (12).

23. Safety jar according to claim 22, **characterised in that** the entrance mouth (17) is tubular shaped, and it is formed by a projecting appendage of a cover (15a) of the safety jar (15).

24. Safety jar according to claim 22 or 23, **characterised in that** the locking ring (32) comprises a cylindrical collar (34) provided with, on the side facing the regulation device (12), an upper edge (36) bent towards the centre of the cylindrical collar (34) itself, in which provided are two opposite recesses (38).

25. Safe jar according to any one of claims 22 to 24, **characterised in that** the entrance mouth (17) is provided at its upper edge with two opposite recesses (42), and having the same dimensions of the two recesses (38) of the locking ring (32).

## Patentansprüche

1. Absaug-Baugruppe zur Verwendung im medizinischen Bereich, dazu geeignet, an eine Vakuumquelle zur Absaugung von Flüssigkeiten, insbesondere organischen Flüssigkeiten, angeschlossen zu werden, der Art, die eine Einstellvorrichtung (12) zum Anschluss an eine Vakuumquelle sowie ein Sicherheitsgefäß (15) zur Aufnahme der Flüssigkeiten umfasst, wobei das Sicherheitsgefäß (15) an der Einstellvorrichtung (12) mit einer Bajonettkupplung (30) abnehmbar befestigt ist, **dadurch gekennzeichnet, dass** die Bajonettkupplung (30) mit einem festen Teil bestehend aus einer Verbindungsöffnung (16) der Einstellvorrichtung (12), und einem beweglichen Teil bestehend aus einem Klemmring (32) versehen ist, der lose drehbar auf einer Eingangsöffnung (17) des Sicherheitsgefäßes (15) angebracht ist.

2. Absaug-Baugruppe nach Anspruch 1, **dadurch gekennzeichnet, dass** die Einstellvorrichtung (12) einen zylinderförmigen Körper umfasst, bestehend aus einem Mantel (23), einer Bodenwand (24) und einem Verschlussdeckel (25).

3. Absaug-Baugruppe nach Anspruch 2, **dadurch gekennzeichnet, dass** die Verbindungsöffnung (16) der Einstellvorrichtung (12) aus einem Rohrstück besteht, das aus der Einstellvorrichtung (12) auskragt.

4. Absaug-Baugruppe nach Anspruch 2, **dadurch gekennzeichnet, dass** die Eingangsöffnung (17) des Sicherheitsgefäßes (15) rohrförmig ist und aus einem Fortsatz besteht, der aus dem Sicherheitsgefäß (15) auskragt.

5. Absaug-Baugruppe nach irgendeinem der vorstehenden Ansprüche von 1 bis 4, **dadurch gekennzeichnet, dass** der Klemmring (32) einen zylinderförmigen Bund (34) umfasst, der auf der Seite, die der Einstellvorrichtung (12) gegenüberliegt, mit einer Kante (36) versehen ist, die zur Mitte des zylinderförmigen Bundes (34) hin gebogen ist, auf dem zwei gegenüberliegende Aussparungen (38) vorgesehen sind.

6. Absaug-Baugruppe nach Anspruch 5, **dadurch gekennzeichnet, dass** die Eingangsöffnung (17) an einem Ende mit zwei gegenüberliegenden Aussparungen (42) versehen ist, die dieselbe Größe wie die beiden Aussparungen (38) des Klemmringes (32) besitzen.

7. Absaug-Baugruppe nach Anspruch 5 oder 6, **dadurch gekennzeichnet, dass** die Verbindungsöffnung (16) der Einstellvorrichtung (12) auf ihrer Außenfläche mit zwei gegenüberliegenden Zähnen (40) versehen ist sowie **dadurch**, dass die Form und Größe der Aussparungen (38) auf der gebogenen Kante des Klemmrings (32) und die der Aussparungen (42) auf der Kante der Eingangsöffnung (17) des Sicherheitsgefäßes im Wesentlichen der Form und Größe der Zähne (40) der Einstellvorrichtung (12) entsprechen, wobei sie insbesondere entsprechend bemessen sind, um die gegenüberliegenden Zähne (40) der Einstellvorrichtung (12) mit begrenztem Freiraum aufzunehmen.

8. Absaug-Baugruppe nach Anspruch 1, **dadurch gekennzeichnet, dass** sie ein ringförmiges Element (44) umfasst, das sich zusammen mit dem Klemmring (32) dreht und zwischen den Klemmring (32) und die Eingangsöffnung (17) des Sicherheitsgefäßes (15) eingefügt ist, um den Klemmring (32) bezüglich der Eingangsöffnung (17) axial zu halten.

9. Absaug-Baugruppe nach Anspruch 8, **dadurch gekennzeichnet, dass** das ringförmige Element (44) mit dem Klemmring (32) durch Einrasten mittels elastischer Verformung von Zähnen (46) verbunden ist, die aus der Außenfläche des ringförmigen Elements (44) in entsprechende Öffnungen (48) hinein auskragen, die auf der zylinderförmigen Oberfläche des Klemmrings (32) vorgesehen sind,

10. Absaug-Baugruppe nach Anspruch 9, **dadurch gekennzeichnet, dass** das ringförmige Element (44) einen im Wesentlichen C-förmigen, eine Aussparung umfassenden Querschnitt besitzt und mit der Eingangsöffnung (17) durch Mittel verbunden ist, die die Aussparung mit einer entsprechenden, runden Auskragung (50) kuppeln, die einstückig aus der Außenfläche der Eingangsöffnung (17) ausragt.

11. Absaug-Baugruppe nach Anspruch 10, **dadurch gekennzeichnet, dass** das ringförmige Element (44) aus zwei Halbringen besteht, die mittels Kupplungs- und Zentrierbolzen miteinander verbunden sind.

12. Absaug-Baugruppe nach Anspruch 1, **dadurch gekennzeichnet, dass** auf der Außenfläche des Sicherheitsgefäßes (15) in der Nähe des Klemmringes (32) Bezugsmarkierurtgen, eine (52) zur Sperrung, die andere (53) hingegen zur Entsperrung, jeweils in einem vorgegebenem Winkelabstand angeordnet sind.

13. Absaug-Baugruppe nach Anspruch 12, **dadurch gekennzeichnet, dass** auf der Außenfläche des Klemmrings (32) eine Anzeigemarkierung (54) vorgesehen ist, die wahlweise auf die Bezugsmarkierungen Sperrung oder Entsperrung ausgerichtet werden kann, die auf der Oberfläche des Sicherheitsgefäßes (15) eingezeichnet sind.

14. Absaug-Baugruppe nach irgendeinem der vorstehenden Ansprüche, **dadurch gekennzeichnet, dass** sie Schnappschlösser zur Stabilisierung der Winkelstellung des Klemmrings in der Sperrungs- bzw. Entsperrungsposition umfasst.

15. Absaug-Baugruppe nach irgendeinem der vorstehenden Ansprüche, **dadurch gekennzeichnet, dass** sie ein Gleitelement (55) umfasst, das als Umschalter zum Öffnen bzw. Schließen eines Absaugkanals (14) dient, der sich in der Einstellvorrichtung (12) erstreckt, wobei das Gleitelement (55) im Wesentlichen rohrförmig und in einem entsprechenden, im Wesentlichen zylinderförmigen Gehäuse (57) gleitbar untergebracht ist, das in der Einstellvorrichtung (12) vorgesehen ist.

16. Absaug-Baugruppe nach Anspruch 15, **dadurch gekennzeichnet, dass** das Gleitelement (55) gegenüberliegende Enden (55a, 55b) aufweist, die so geformt sind, dass sie einen Knopf bilden, wobei es so bemessen ist, dass nach Aufnahme im Gehäuse (57) der Einstellvorrichtung (12) nur eines der beiden Enden (55a, 55b) aus der Einstellvorrichtung (12) auskragt.

17. Absaug-Baugruppe nach Anspruch 15 oder 16, **dadurch gekennzeichnet, dass** das Gleitelement (55) aus zwei Teilen besteht, insbesondere aus zwei im Wesentlichen rohrförmigen Teilen, deren jeweilige benachbarte Enden durch Schnappkupplung verbunden sind.

18. Absaug-Baugruppe nach Anspruch 17, **dadurch gekennzeichnet, dass** ein erster Teil des Gleitelementes (55) mit einem Endabschnitt versehen ist, der zwei gegenüberliegende Frontzähne (59a) trägt, während ein zweiter Teil des Gleitelements mit einem Endabschnitt (59b) versehen ist, der entsprechend geformt ist, um ein Rohrstück zu bilden, in dem die Frontzähne (59a) des anderen Teils mittels elastischer Verbindung eingepasst sind.

19. Absaug-Baugruppe nach irgendeinem der vorstehenden Ansprüche, **dadurch gekennzeichnet, dass** sie außerdem ein Vakuummessgerät (13), das mit der Einstellvorrichtung (12) verbunden ist sowie Mittel zur Verbindung des Vakuummessgeräts (13) mit der Einstellvorrichtung (12) umfasst, wobei die Mittel einen Einschub (60) umfassen, der quer in der Einstellvorrichtung (12) bewegt und in einem Gehäuse (62) untergebracht werden kann, das in der Einstellvorrichtung (12) hierfür vorgesehen ist.

20. Absaug-Baugruppe nach Anspruch 19, **dadurch gekennzeichnet, dass** der Einschub (60) mittels eines Gewindeanschlusses (61) an der Unterseite des Vakuummessgeräts (13) befestigt ist.

21. Absaug-Baugruppe nach Anspruch 20, **dadurch gekennzeichnet, dass** der Einschub (60) aus einem Körper besteht, der im Wesentlichen geformt ist, um einen Block zu bilden, der geführt in das Gehäuse (62) eingepasst werden kann.

22. Sicherheitsgefäß zur Aufnahme organischer Flüssigkeiten für eine Absaug-Baugruppe zur Verwendung im medizinischen Bereich, umfassend eine Eingangsöffnung (17), die dazu bestimmt ist, abnehmbar an einer Vakuumeinstellvorrichtung (12) befestigt zu werden, sowie eine Verbindungsöffnung (18), die dazu bestimmt ist, an die abzusaugenden Flüssigkeiten angeschlossen zu werden, wobei der Klemmring (32) lose drehbar auf der Eingangsöffnung (17) angebracht ist und einen beweglichen Teil einer Bajonettkupplung (30) bildet, der auf einem entsprechenden festen Teil der Bajonettkupplung befestigt wird, der mit der Einstellvorrichtung (12) verbunden ist.

23. Sicherheitsgefäß nach Anspruch 22, **dadurch gekennzeichnet, dass** die Eingangsöffnung (17) rohrförmig ist und aus einem auskragenden Fortsatz eines Deckels (15a) des Sicherheitsgefäßes (15) gebildet wird.

24. Sicherheitsgefäß nach Anspruch 22 oder 23, **dadurch gekennzeichnet, dass** der Klemmring (32) einen zylinderförmigen Bund (34) umfasst, der auf der der Einstellvorrichtung (12) gegenüberliegenden Seite mit einer oberen Kante (36) versehen ist, die zur Mitte des zylinderförmigen Bundes (34) hin gebogen ist, auf dem zwei gegenüberliegende Aussparungen (38) vorgesehen sind.

25. Sicherheitsgefäß nach irgendeinem der Ansprüche 22 bis 24, **dadurch gekennzeichnet, dass** die Eingangsöffnung (17) an ihrer oberen Kante mit zwei gegenüberliegenden Aussparungen (42) versehen ist, die dieselbe Größe wie die beiden Aussparungen (38) des Klemmrings (32) besitzen.

## Revendications

1. Ensemble d'aspiration pour l'utilisation dans le domaine médical, adapté pour être connecté à une source de vide pour l'aspiration de fluides, en particulier des fluides organiques, du type comprenant un dispositif de régulation (12) destiné à être connecté à une source de vide et un récipient de sécurité (15) pour recueillir des fluides, dans lequel ledit récipient de sécurité (15) est fixé de manière amovible au dispositif de régulation (12) par un accouplement à baïonnette (30), **caractérisé en ce que** le verrouillage à baïonnette (30) est pourvu d'une partie fixe formée par une embouchure de connexion (16) du dispositif de régulation (12) et d'une partie mobile formée par une bague de verrouillage (32) disposée de manière libre en rotation sur une embouchure d'entrée (17) du récipient de sécurité (15).

2. Ensemble d'aspiration selon la revendication 1, **caractérisé en ce que** le dispositif de régulation (12) comprend un corps de forme cylindrique, formé par un manchon (23), une paroi de fond (24) et un couvercle de fermeture (25).

3. Ensemble d'aspiration selon la revendication 2, **caractérisé en ce que** l'embouchure de connexion (16) du dispositif de régulation (12) est formée par un tronçon de tube faisant saillie du dispositif de régulation (12).

4. Ensemble d'aspiration selon la revendication 2, **caractérisé en ce que** l'embouchure d'entrée (17) du récipient de sécurité (15) est de forme tubulaire et elle est formée par un appendice faisant saillie du récipient de sécurité (15).

5. Ensemble d'aspiration selon l'une quelconque des revendications précédentes 1 à 4, **caractérisé en ce que** la bague de verrouillage (32) comprend un collier cylindrique (34) pourvu, sur le côté faisant face au dispositif de régulation (12), d'un bord (36) plié vers le centre du collier cylindrique (34), sur lequel sont prédisposés deux évidements opposés (38).

6. Ensemble d'aspiration selon la revendication 5, **caractérisé en ce que** l'embouchure d'entrée (17) est pourvue à une extrémité de deux évidements opposés (42), et ayant les mêmes dimensions que les deux évidements (38) de la bague de verrouillage (32).

7. Ensemble d'aspiration selon la revendication 5 ou 6, **caractérisé en ce que** l'embouchure de connexion (16) du dispositif de régulation (12) est pourvue sur sa surface externe de deux dents opposées (40), et **en ce qu'**à la fois les évidements (38) prévus sur le bord plié de la bague de verrouillage (32) et les évidements (42) prévus sur le bord de l'embouchure d'entrée (17) du récipient de sécurité ont des formes et dimensions correspondant sensiblement aux dents (40) du dispositif de régulation (12), et, en particulier, **en ce qu'**ils sont dimensionnés pour recevoir avec un jeu limité les dents opposées (40) du dispositif de régulation (12).

8. Ensemble d'aspiration selon la revendication 1, **caractérisé en ce qu'**il comprend un élément annulaire (44), qui tourne conjointement à la bague de verrouillage (32) et est interposé entre la bague de verrouillage (32) et l'embouchure d'entrée (17) du récipient de sécurité (15), pour maintenir axialement la bague de verrouillage (32) par rapport à l'embouchure d'entrée (17).

9. Ensemble d'aspiration selon la revendication 8, **caractérisé en ce que** l'élément annulaire (44) est connecté à la bague de verrouillage (32) par encliquetage, obtenu par le biais de la déformation élastique de dents (46) faisant saillie de la surface externe de l'élément annulaire (44) dans des ouvertures correspondantes (48) prévues sur la surface cylindrique de la bague de verrouillage (32),

10. Ensemble d'aspiration selon la revendication 9, **caractérisé en ce que** l'élément annulaire (44) a une section transversale sensiblement en forme de C comprenant un évidement et il est connecté à l'embouchure d'entrée (17) par le biais de l'accouplement de l'évidement avec une saillie circulaire correspondante (50) faisant saillie intégralement de la surface externe de l'embouchure d'entrée (17).

11. Ensemble d'aspiration selon la revendication 10, **caractérisé en ce que** l'élément annulaire (44) est formé par deux demi-anneaux unis l'un avec l'autre au moyen de goupilles de centrage et d'accouplement.

12. Ensemble d'aspiration selon la revendication 1, **caractérisé en ce que**, sur la surface externe du récipient de sécurité (15) indiquée à proximité de la bague de verrouillage (32), se trouvent des marques de référence, l'une (52) pour le verrouillage, l'autre (53) pour le déverrouillage, disposées respectivement à une distance angulaire
prédéterminée.

13. Ensemble d'aspiration selon la revendication 12, **caractérisé en ce que**, sur la surface externe de la bague de verrouillage (32), est prévue une marque d'indicateur (54) destinée à être disposée alternativement au niveau des marques de référence, verrouillage et déverrouillage, tracées sur la surface du récipient de sécurité (15).

14. Ensemble d'aspiration selon l'une quelconque des revendications précédentes, **caractérisé en ce qu'**il comprend des verrouillages à encliquetage pour stabiliser la position angulaire de la bague de verrouillage au niveau des deux positions de verrouillage et déverrouillage.

15. Ensemble d'aspiration selon l'une quelconque des revendications précédentes, **caractérisé en ce qu'**il comprend un élément coulissant (55) agissant comme un commutateur destiné respectivement à ouvrir et fermer une voie d'aspiration (14) s'étendant dans le dispositif de régulation (12), ledit élément coulissant (55) étant de forme sensiblement tubulaire et étant logé de manière coulissante dans un logement de forme sensiblement cylindrique correspondante (57) prédisposé dans le dispositif de régulation (12).

16. Ensemble d'aspiration selon la revendication 15, **caractérisé en ce que** l'élément coulissant (55) a des extrémités opposées (55a, 55b) profilées pour former un bouton, et **en ce qu'**il est dimensionné de manière que, une fois disposé dans le logement (57) du dispositif de régulation (12), seule une des deux extrémités (55a, 55b) fasse saillie du dispositif de régulation (12).

17. Ensemble d'aspiration selon la revendication 15 ou 16, **caractérisé en ce que** l'élément coulissant (55) est réalisé en deux parties, et en particulier deux parties de forme sensiblement tubulaire, et ayant des extrémités adjacentes respectives connectées par accouplement à encliquetage.

18. Ensemble d'aspiration selon la revendication 17, **caractérisé en ce qu'**une première partie de l'élément coulissant (55) est pourvue d'une section d'extrémité portant deux dents frontales opposées (59a) et une deuxième partie de l'élément coulissant est pourvue d'une section d'extrémité (59b) profilée pour former un tronçon de tube, à l'intérieur duquel les dents frontales (59a) de l'autre partie sont ajustées au moyen d'un engagement élastique.

19. Ensemble d'aspiration selon l'une quelconque des revendications précédentes, **caractérisé en ce qu'**il comprend en outre un manomètre à vide (13) connecté au dispositif de régulation (12) et des moyens pour connecter le manomètre à vide (13) au dispositif de régulation (12), dans lequel lesdits moyens comprennent un tiroir (60) mobile transversalement dans le dispositif de régulation (12) et peuvent être logés dans un logement (62) prévu dans le dispositif de régulation (12).

20. Ensemble d'aspiration selon la revendication 19, **caractérisé en ce que** le tiroir (60) est fixé au moyen d'une connexion filetée (61) à un côté inférieur du manomètre à vide (13).

21. Ensemble d'aspiration selon la revendication 20, **caractérisé en ce que** le tiroir (60) est formé par un corps profilé sensiblement pour former un bloc qui peut être inséré de manière guidée dans le logement (62).

22. Récipient de sécurité pour recueillir des fluides organiques pour un ensemble d'aspiration destiné au domaine médical, comprenant une embouchure d'entrée (17), destinée à être fixée de manière amovible à un dispositif de régulation du vide (12) et une embouchure de connexion (18) destinée à être connectée aux fluides à aspirer, **caractérisé en ce qu'**il comprend une bague de verrouillage (32) pour la fixation de manière amovible au dispositif de régulation (12), ladite bague de verrouillage (32) étant disposée de manière libre en rotation sur l'embouchure d'entrée (17) et constituant une partie mobile d'un accouplement à baïonnette (30) destinée à être fixée sur une partie fixe correspondante de l'accouplement à baïonnette, associée au dispositif de régulation (12).

23. Récipient de sécurité selon la revendication 22, **caractérisé en ce que** l'embouchure d'entrée (17) est de forme tubulaire et elle est formée par un appendice en saillie d'un couvercle (15a) du récipient de sécurité (15).

24. Récipient de sécurité selon la revendication 22 ou 23, **caractérisé en ce que** la bague de verrouillage (32) comprend un collier cylindrique (34) pourvu, sur le côté faisant face au dispositif de régulation (12), d'un bord supérieur (36) plié vers le centre du collier cylindrique (34), sur lequel sont prédisposés deux évidements opposés (38).

25. Récipient de sécurité selon l'une quelconque des revendications 22 à 24, **caractérisé en ce que** l'embouchure d'entrée (17) est pourvue au niveau de son bord supérieur de deux évidements opposés (42), et ayant les mêmes dimensions que les deux évidements (38) de la bague de verrouillage (32).
